# EUROPÄISCHE PATENTSCHRIFT

(11) **EP 1 129 696 B1**
(45) Veröffentlichungstag und Bekanntmachung des Hinweises auf die Patenterteilung: **15.11.2006**
(21) Anmeldenummer: 01103040.0
(22) Anmeldetag: 09.02.2001
(51) Int. Cl.: A61K 8/35, A61K 8/37, A61K 8/40, A61Q 17/04

(54) **Stabilisierung oxidations- und/oder UV-empfindlicher Wirkstoffe**
Stabilization of oxidation- and/or UV- sensitive active agents
Stabilisation de composés sensibles à l'oxydation et au rayonnement UV

(30) Priorität: 25.02.2000 DE 10008895
(43) Veröffentlichungstag der Anmeldung: 05.09.2001
(73) Patentinhaber: Beiersdorf AG, 20253 Hamburg (DE)
(72) Erfinder: Gers-Barlag, Heinrich, Dr., 25495 Kummerfeld (DE); Wendel, Volker, Dr., 20255 Hamburg (DE); Grundt, Wiebke, 21244 Buchholz (DE)

(56) Entgegenhaltungen:
- EP-A- 0 514 491
- FR-A- 2 801 210
- US-A- 5 547 659
- US-A- 5 993 789
- BONDA, CRAIG ET AL: "A new photostabilizer for full spectrum sunscreens" COSMET. TOILETRIES (2000), 115(6), 37-45 , XP001010090

## Beschreibung

Die vorliegende Erfindung betrifft kosmetische Lichtschutzformulierungen und Formulierungen mit UV-empfindlichen Lichtschutzfiltersubstanzen, die durch den Einsatz einer bestimmten Stoffkombination stabilisiert werden.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Eine vorteilhafte Lichtschutzfiltersubstanz ist beispielsweise das 2-Ethylhexyl-p-methoxy-cinnamat (4-Methoxyzimtsäure-2'-ethylhexylhester), welches von Givaudan unter der Bezeichnung Parsol® MCX erhältlich ist und sich durch folgende Struktur auszeichnet:

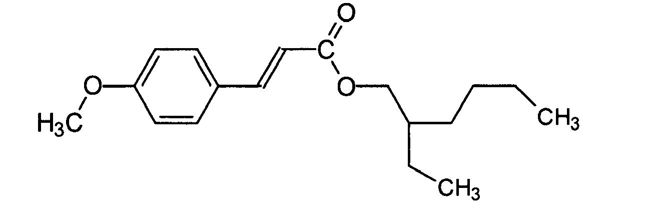

Der Hauptnachteil von 2-Ethylhexyl-p-methoxy-cinnamat ist eine gewisse Instabilität gegenüber UV-Strahlung, so daß Zubereitungen mit einem Gehalt an dieser Substanz zweckmäßigerweise auch bestimmte UV-Stabilisatoren enthalten sollten.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Bekannte und vorteilhafte Lichtschutzfiltersubstanzen, die insbesondere auch gegenüber UV-A-Strahlung Schutz gewähren, sind Dibenzoylmethanderivate, insbesondere das 4-(tert.-Butyl)-4'-methoxydibenzoylmethan, welches sich durch die Struktur 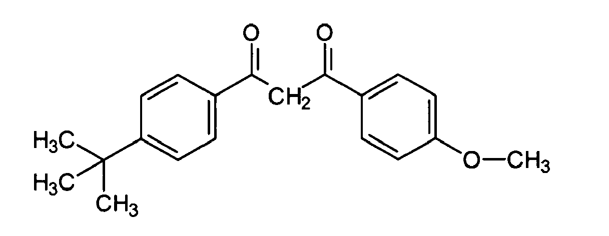 auszeichnet und von Givaudan unter der Marke Parsol® 1789 verkauft wird.

Der Hauptnachteil auch dieser Substanz ist eine gewisse Instabilität gegenüber UV-Strahlung. Die photochemische Zersetzung aller im UV-Bereich absorbierenden Dibenzoylmethanderivate folgt einer Norrish-Typ-I-Acylspaltung. Diese wird am Beispiel von 4-(tert.-Butyl)-4'-methoxydibenzoylmethan im nachfolgenden Reaktionsschema dargestellt:

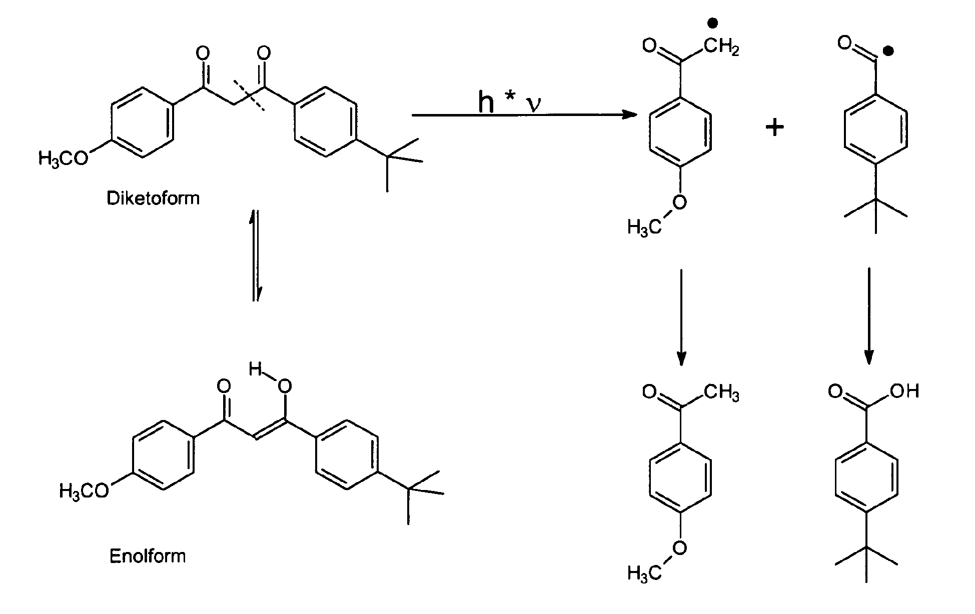

Für Gemische aus den bereits genannten Cinnamaten und Dibenzoylmethanen gilt im Prinzip das bereits gesagte. Im Gegensatz zu manchen Lichtschutzfilterkombinationen, die sich durch erhöhte Stabilität gegenüber den jeweiligen Einzelsubstanzen auszeichnen, wird im allgemeinen für Gemische aus Cinnamaten und Dibenzoylmethanen sogar eine stärkere Destabilisierung gegenüber UV-Licht beobachtet als für die jeweiligen Einzelsubstanzen. Dies ist insbesondere bei Gemischen aus 2-Ethylhexyl-p-methoxy-cinnamat und 4-(tert.-Butyl)-4'-methoxydibenzoylmethan der Fall. Mit einiger Sicherheit reagieren diese beiden Komponenten unter UV-Einfluß zu inaktiven Produkten und stehen für die UV-Absorption nicht mehr zur Verfügung.

Als mögliche Lösungen dieses Problems ist verschiedentlich vorgeschlagen worden, die Wirkstoffe durch Zugabe eines oder mehrere Stabilisatoren, z. B. Antioxidantien, vor dem Abbau zu bewahren oder sie in Form von stabileren Derivaten einzusetzen.

Auch 2-Ethylhexyl-2-cyano-3,3-diphenylacrylat (INCI: Octocrylene) - eine bei Raumtemperatur flüssige UV-Filtersubstanz - soll eine stabilisierende Wikrung auf 4-(tert.-Butyl)-4'-methoxydibenzoylmethan haben. 2-Ethylhexyl-2-cyano-3,3-diphenylacrylat zeichnet sich durch folgende Struktur aus: 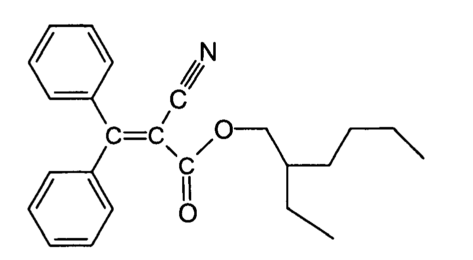 und ist beispielsweise von BASF unter der Bezeichnung Uvinul^{®} N 539 erhältlich.

Eine ähnliche Wirkung ist auch für Diethylhexylnaphthalat, welches durch die Strukturformel 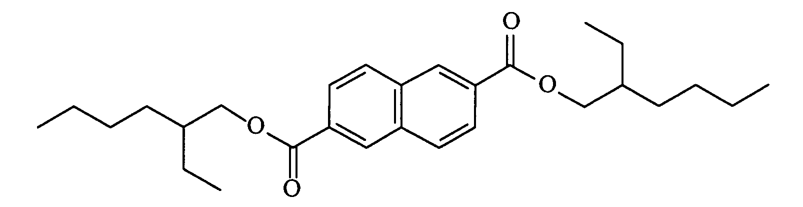 wiedergegeben wird, bereits beschrieben worden.

Allerdings bleibt in allen Fällen die erzielte Wirkung deutlich hinter der erhofften zurück.

US 5,993,789 beschreibt die Verwendung von Naphthalendicarbonsäure - Polyestern bzw. Diestern zur Erhöhung der Photostabilität von Dibenzoylmethan-Derivaten, insbesondere von Avobenzen, in Sonnenschutzmitteln. Diethylhexylnaphthalat wird als möglicher Naphthalendicarbonsäure - Diester aufgeführt.

Der Stand der Technik konnte keinerlei Hinweise geben, welche Rückschlüsse auf die erfindungsgemäßen Zubereitungen erlaubt hätten.

Die Aufgabe der vorliegenden Erfindung war, den Nachteilen des Standes der Technik abzuhelfen und die Stabilität von oxidationsempfindlichen bzw. UV-empfindlichen Lichtschutzfiltersubstanzen zu erhöhen sowie stabile Zubereitungen mit oxidationsempfindlichen bzw. UV-empfindlichen Lichtschutzfiltersubstanzen zu schaffen, deren Wirksamkeit über einen langen Zeitraum erhalten bleibt.

Es war überraschend und für den Fachmann in keiner Weise vorhersehbar, daß eine kosmetische oder dermatologische Formulierung mit mindestens einer oxidations- und/oder UV-empfindlichen Lichtschutzfiltersubstanz gewählt aus 4-(tert.)-Butyl)-4'-methoxydibenzoyl-methan oder 2-Ethylhexyl-p-methoxy-cinnamat, dadurch gekennzeichnet, dass sie eine synergistische Stoffkombination aus 2-Ethylhexyl-2-cyano-3,3-diphenylacrylat und Diethylhexylnaphthalat enthält und dadurch gekennzeichnet, dass der Gehalt an 2-Ethylhexyl-2-cyano-3,3-diphenylacrylat kleiner als 1 Gew.-% ist, bezogen auf das Gesamtgewicht der Formulierung
b) den Nachteilen des Standes der Technik abhelfen würden.

Die erfindungsgemäßen Kombinationen wirken ertaunlicherweise synergistisch, also überadditiv in bezug auf die Einzelkomponenten. Durch Kombination der erfindungsgemäßen Einzelsubstanzen werden der oder die oxidations- und/oder UV-empfindlichen Lichtschutzfiltersubstanzen, insbesonderes in kosmetischen oder dermatologischen Formulierungen in hervorragender Weise gegen die durch UV-Strahlung induzierte Zersetzung geschützt.

Bei Befolgen der erfindungsgemäßen Lehre sind Lichtschutzzubereitungen erhältlich, welche höhere Stabilität, insbesondere Stabilität gegen Zersetzung unter dem Einfluß von Licht, ganz besonders UV-Licht, aufweisen, als der Stand der Technik hätte erwarten lassen. Insbesondere die Stabilität von 4-(tert.-Butyl)-4'-methoxydibenzoylmethan gegen die Zersetzung unter UV-Licht wird drastisch erhöht. Ganz besonders erstaunlich war, daß die Erhöhung der Stabilität von 4-(tert.-Butyl)-4'-methoxydibenzoylmethan gleichermaßen erfolgt, wenn diese in polaren aber auch unpolaren Ölkomponenten gelöst vorliegt.

Die Gesamtmenge an 4-(tert.-Butyl)-4'-methoxydibenzoylmethan in den fertigen kosmetischen oder dermatologischen Zubereitungen wird vorteilhaft aus dem Bereich von 0,1 bis 10,0 Gew.-%, bevorzugt 0,5 bis 6,0 Gew.-% gewählt, bezogen auf das Gesamtgewicht der Zubereitungen.

Die Gesamtmenge an Diethylhexylnaphthalat in den fertigen kosmetischen oder dermatologischen Zubereitungen wird vorteilhaft aus dem Bereich von 4 bis 16 Gew.-%, bevorzugt 6 bis 14 Gew.-% gewählt, insbesondere bevorzugt 6 bis 10 Gew.-% gewählt, jeweils bezogen auf das Gesamtgewicht der Zubereitungen.

Die erfindungsgemäßen Stoffkominationen können vorteilhaft in kosmetischen und/oder dermatologischen Formulierungen eingesetzt werden, welche wie üblich zusammengesetzt sein können und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Es ist vorteilhaft, das Verhältnis von 2-Ethylhexyl-2-cyano-3,3-diphenylacrylat zu der oder den oxidations- und/oder UV-empfindlichen Lichtschutzfiltersubstanzen aus dem Bereich von 1 : 1 bis 0,1 : 1 zu wählen.

Kosmetische und dermatologische Zubereitungen, welche die erfindungsgemäßen Stoffkominationen enthalten, können in verschiedenen Formen vorliegen. So können sie z. B. eine Lösung, eine O/W-Emulsion oder O/W-Mikroemulsion, eine W/O-Emulsion oder W/O-Mikroemulsion, eine Pickering-Emulsion, eine multiple Emulsion (z. B. eine W/O/W-Emulsion), eine sprühbare Emulsion, eine Hydrodispersion, ein Aerosol, einen Schaum oder auch einen Stift darstellen.

Zur Anwendung werden die kosmetischen und dermatologischen Zubereitungen in der für Kosmetika üblichen Weise auf die Haut und/oder die Haare in ausreichender Menge aufgebracht.

Günstig sind auch solche kosmetischen und dermatologischen Zubereitungen, die in Form eines Sonnenschutzmittels vorliegen. Vorzugsweise enthalten diese neben den erfindungsgemäßen Stoffkombinationen zusätzlich mindestens eine weitere UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten Zubereitungen enthaltend die erfindungsgemäße Stoffkombination ferner Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte weitere UV-A-Filtersubstanzen im Sinne der vorliegenden Erfindung sind die Phenylen-1,4-bis-(2-benzimidazyl)-3,3'-5,5'-tetrasulfonsäure: 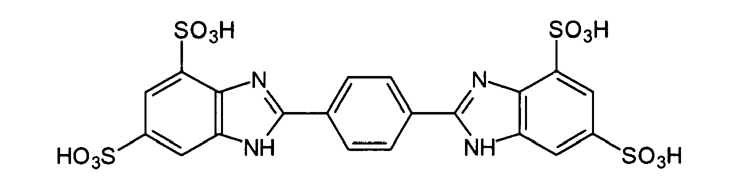 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 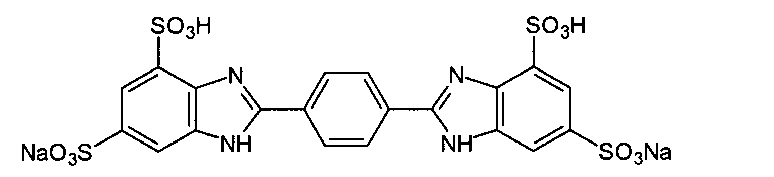 sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

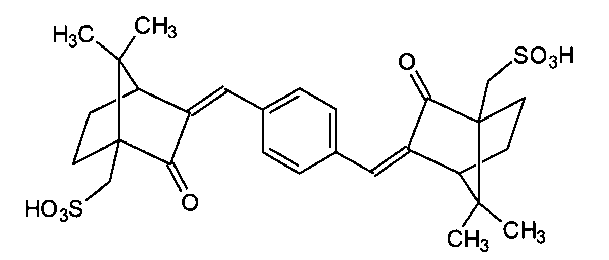

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 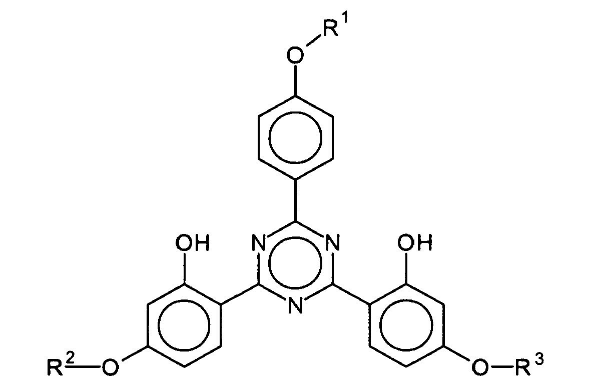 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen beschrieben, welche das Strukturmotiv 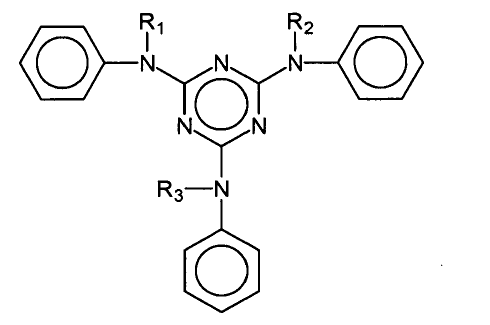 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 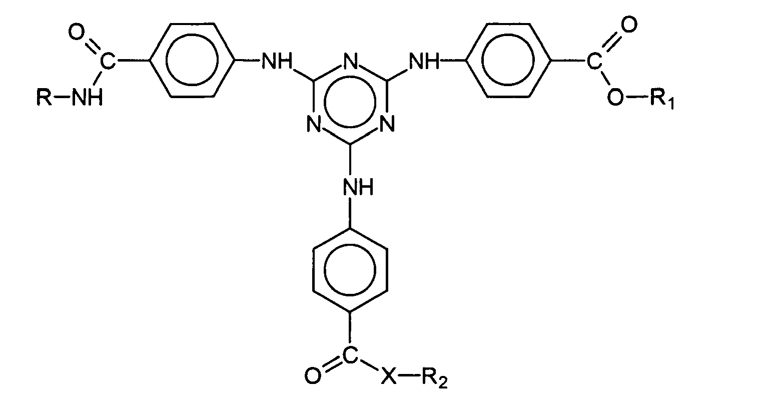 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 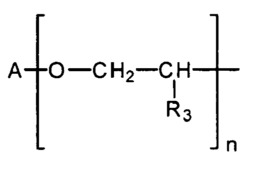 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 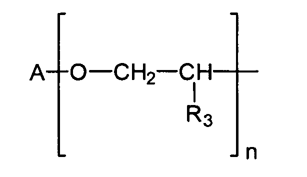 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 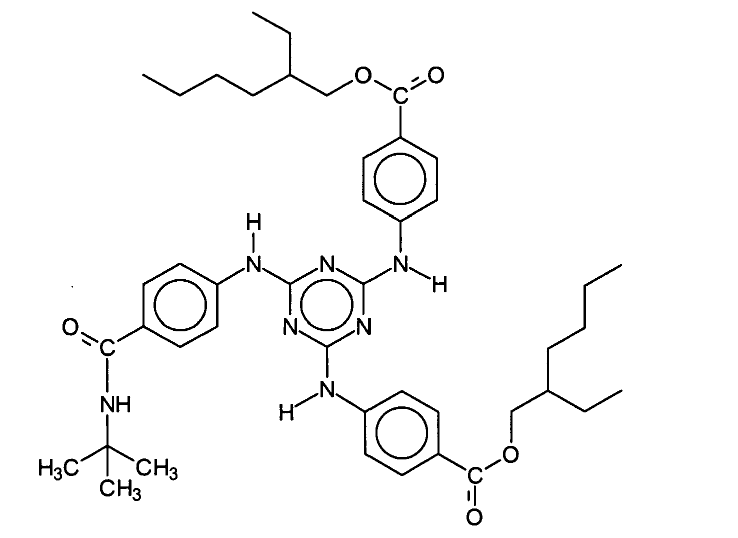 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel 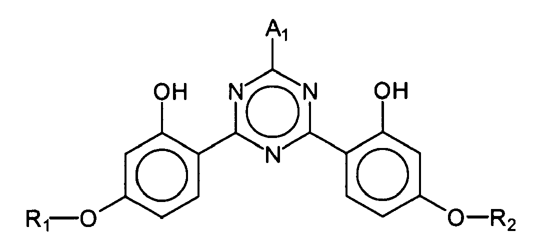 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 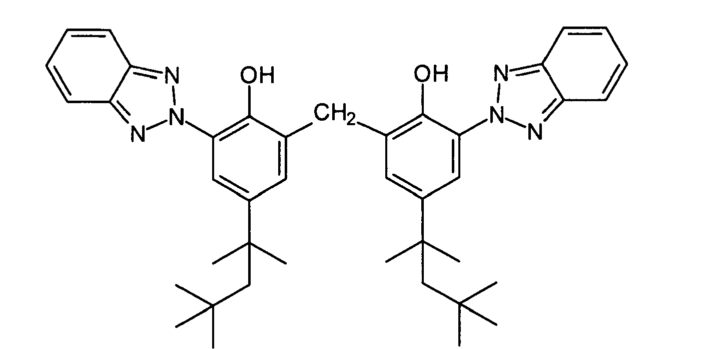 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 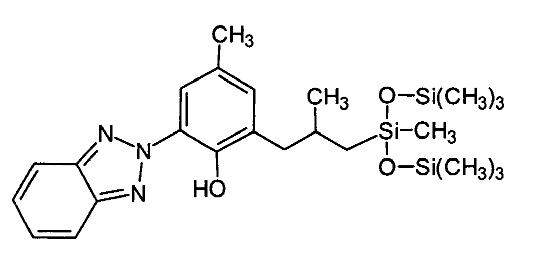 gekennzeichnet ist.

Die UV-B-Filter können öllöslich oder wasserlöslich sein. Vorteilhafte öllösliche UV-B-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die kosmetischen und dermatologischen Zubereitungen enthaltend die erfindungsgemäße Stoffkombination können ferner kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfs- und/oder Trägerstoffen und Parfüm können in Abhängigkeit von der Art des jeweiligen Produktes vom Fachmann durch einfaches Ausprobieren leicht ermittelt werden.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die Zubereitungen mit einem Gehalt an einem oder mehreren Antioxidantien zu versehen, wenn die Zubereitungen einem anderen Zweck dienen sollen, z. B. als Sonnenschutzmittel.

Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Ölphase der Formulierungen, welche die erfindungsgemäße Stoffkombination enthalten, wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Caprylic/Caprictriglycerid, Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Ferner kann die Ölphase vorteilhaft gewählt werden aus der Gruppe der Dialkylether, vorteilhaft ist z. B. Dicaprylylether.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Caprylat/Caprat, C₁₂₋₁₃-Alkyllactat, Di-C_{12/13}-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft sind ferner z. B. natürliche Wachse tierischen und pflanzlichen Ursprungs, wie beispielsweise Bienenwachs, Chinawachs, Hummelwachs und andere Insektenwachse sowie Sheabutter.

Die wäßrige Phase der Zubereitungen enthaltend die erfindungsgemäße Stoffkombination enthält gegebenenfalls vorteilhaft:
Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Zahlenwerte in den nachfolgenden Referenz-Beispielen bedeuten Gewichtsprozente, bezogen auf das Gesamtgewicht der jeweiligen Zubereitungen.

### Referenz-Beispiele:

| | **Beispiel 1** | **Beispiel 2** | **Beispiel 3** |
|---|---|---|---|
| | O/W | O/W1 | O/W2 |
| Stearinsäure | 1,50 | - | - |
| Glycerinmonostearat | 3,00 | - | - |
| Sorbitanstearat | - | 2,00 | 3,00 |
| Polyglyceryl-3-methylglucose | - | 4,00 | 1,50 |
| Distearat | | | |
| Caprylic/Capric Triglycerid | - | 5,00 | - |
| Octyldodecanol | - | 5,00 | - |
| Dicaprylyl Ether | - | 5,00 | - |
| Dimethicon | 2,00 | - | - |
| Phenyltrimethicon | 2,00 | - | - |
| Vitamin E-Acetat | 0,50 | - | 0,50 |
| Dioctylbutamidotriazon | 3,00 | - | - |
| Aniso Triazin | - | - | 3,00 |
| Octocrylen | 1,50 | 1,90 | 2,90 |
| Octylsalicylat | 5,00 | - | - |
| Octyltriazon | - | 2,00 | - |
| Methylbenzylidencampher | - | - | 4,00 |
| Butyl Methoxydibenzoyl Methan | 2,00 | 2,00 | 3,00 |
| Eusolex T2000 ®¹ | 1,00 | - | - |
| Hallbrite TQ ®² | 4,00 | 8,00 | 7,00 |
| Konservierung | 0,50 | 0,50 | 0,50 |
| Glycerin | 3,00 | 3,00 | 10,00 |
| Xanthan Gum | 0,30 | 0,50 | - |
| Pemulen TR1 ® | - | - | 0,10 |
| Phenylbenzimidazol Sulfonsäure | - | 2,00 | 2,00 |
| Natronlauge 45% | 0,50 | - | 1,20 |
| Wasser | ad 100,00 | ad 100,00 | ad 100,00 |

| | | | |
|---|---|---|---|
| ¹mit Simethicone und Alumina beschichtete Titandioxidpigmente ² Diethylhexylnaphthalat | | | |

| | **Beispiel 4** | **Beispiel 5** | **Beispiel 6** |
|---|---|---|---|
| | W/O 1 | W/O 2 | W/O 3 |
| Polyglyceryl-2 | 5,00 | - | - |
| Dipolyhydroxystearat | | | |
| Cetyl Dimethicon Copolyol | - | - | 5,00 |
| PEG-30 Dipolyhydroxystearat | - | 4,00 | - |
| Dimethicon | 2,00 | - | 5,00 |
| Phenyltrimethicon | - | 5,00 | 3,00 |
| Vitamin E-Acetat | - | 0,50 | - |
| Dioctylbutamidotriazon | 3,00 | - | 5,00 |
| Aniso Triazin | 2,00 | 5,00 | 2,00 |
| Octocrylen | 3, 50 | 1,90 | 1,00 |
| Octylsalicylat | 5,00 | - | 5,00 |
| Octylmethoxycinnamat | 8,00 | 10,00 | 10,00 |
| Octyltriazon | 2,00 | - | 1,00 |
| Methylbenzylidencampher | - | - | 4,00 |
| Butyl Methoxydibenzoyl Methan | 4,00 | 2,00 | - |
| Eusolex T2000 ®¹ | - | - | 2,00 |
| Aerosil R972 ®² | - | 0,50 | - |
| Hallbrite TQ ®³ | 5,00 | 4,00 | 6,00 |
| Konservierung | 0,50 | 0,50 | 0,50 |
| Glycerin | 5,00 | 10,00 | 5,00 |
| MgSO₄ | 1,00 | 1,00 | - |
| NaCl | - | - | 1,00 |
| Phenylbenzimidazol Sulfonsäure | - | - | 4,00 |
| Natronlauge 45% | - | - | 1,30 |
| Wasser | ad 100,00 | ad 100,00 | ad 100,00 |

| | | | |
|---|---|---|---|
| ¹mit Simethicone und Alumina beschichtete Titandioxidpigmente ² wasserabweisend beschichtete Siliciumdioxidpartikel ³ Diethylhexylnaphthalat | | | |

| | **Beispiel 7** | **Beispiel 8** | **Beispiel 9** |
|---|---|---|---|
| | W/O-Pickering-emulsion | Spray | Spray |
| Glycerinmonostearat | - | 4,00 | - |
| Glycerinmonostearat SE | - | - | 4,50 |
| Ceteareth-20 | - | - | 1,00 |
| Ceteareth-12 | - | 1,50 | - |
| Dimethicon | - | - | 2,00 |
| Phenyltrimethicon | 5,00 | - | - |
| Vitamin E-Acetat | 0,50 | - | - |
| Dioctylbutamidotriazon | 5,00 | - | 2,00 |
| Aniso Triazin | 5,00 | 2,00 | - |
| Octocrylen | 2,90 | 2,90 | 1,50 |
| Octylsalicylat | 5,00 | - | 2,00 |
| Octylmethoxycinnamat | 10,00 | 5,00 | - |
| Octyltriazon | - | 1,00 | - |
| Butylmethoxydibenzoylmethan | 3,00 | 3,00 | 2,00 |
| Eusolex T2000 ®¹ | 5,00 | - | - |
| Aerosil R972 ®² | 1,00 | - | - |
| Hallbrite TQ ®³ | 6,00 | 4,00 | 4,00 |
| Konservierung | 0,50 | 0,50 | 0,50 |
| Glycerin | 3,00 | 10,00 | 5,00 |
| Phenylbenzimidazol | - | 1,00 | - |
| Sulfonsäure | | | |
| Natronlauge 45% | - | 0,40 | - |
| Wasser | ad 100 | ad 100 | ad 100 |

| | | | |
|---|---|---|---|
| ¹ mit Simethicone und Alumina beschichtete Titandioxidpigmente ² wasserabweisend beschichtete Siliciumdioxidpartikel ³ Diethylhexylnaphthalat | | | |

## Patentansprüche

1. Kosmetische oder dermatologische Formulierung mit mindestens einer oxidations- und/oder UV-empfindlichen Lichtschutzfiltersubstanz gewählt aus 4-(tert.)-Butyl)-4'-methoxydibenzoyl-methan oder 2-Ethylhexyl-p-methoxy-cinnamat, **dadurch gekennzeichnet, dass** sie eine synergistische Stoffkombination aus 2-Ethylhexyl-2-cyano-3,3-diphenylacrylat und Diethylhexylnaphthalat enthält und **dadurch gekennzeichnet, dass** der Gehalt an 2-Ethylhexyl-2-cyano-3,3-diphenylacrylat kleiner als 1 Gew.-% ist, bezogen auf das Gesamtgewicht der Formulierung.

2. Formulierung nach Anspruch 1, **dadurch gekennzeichnet, dass** das Verhältnis von 2-Ethylhexyl-2-cyano-3,3-diphenylacrylat zu der oder den oxidations- und/oder UV-empfindlichen Lichtschutzfiltersubstanzen aus dem Bereich von 1 : 1 bis 0,1 : 1 gewählt wird.

## Claims

1. Cosmetic or dermatological formulation with at least one oxidation- and/or UV-sensitive photoprotective filter substance chosen from 4-(tert-butyl)-4'-methoxydibenzoylmethane or 2-ethylhexyl p-methoxycinnamate, **characterized in that** it comprises a synergistic substance combination of 2-ethylhexyl 2-cyano-3,3-diphenylacrylate and diethylhexyl naphthalate and **characterized in that** the content of 2-ethylhexyl 2-cyano-3,3-diphenylacrylate is less than 1% by weight, based on the total weight of the formulation.

2. Formulation according to Claim 1, **characterized in that** the ratio of 2-ethylhexyl 2-cyano-3,3-diphenylacrylate to the oxidation- and/or UV-sensitive photoprotective filter substance(s) is chosen from the range from 1:1 to 0.1:1.

## Revendications

1. Formulation cosmétique ou dermatologique comprenant au moins une substance filtrante photoprotectrice sensible à l'oxydation et au rayonnement UV, choisie parmi le 4-(tert-butyl)-4'-méthoxydibenzoyl-méthane ou le p-méthoxycinnamate de 2-éthylhexyle, **caractérisée en ce qu'**elle comprend une combinaison de substances synergique de 2-éthylhexil-2-cyano-3,3-diphénylacrylate et de naphtalate de diéthylhexyle, et **caractérisée en ce que** la teneur en 2-éthylhexyl-2-cyano-3,3-diphénylacrylate est inférieure à 1 % en poids, par rapport au poids total de la formulation.

2. Formulation selon la revendication 1, **caractérisée en ce que** le rapport entre 2-éthylhexyl-2-cyano-3,3-diphénylacrylate et la ou les substance(s) filtrante(s) photoprotectrice(s) sensible(s) à l'oxydation et/ou au rayonnement UV est choisi dans la plage de 1:1 à 0,1:1.
